# EUROPEAN PATENT SPECIFICATION

(11) **EP 0 751 930 B1**
(45) Date of publication and mention of the grant of the patent: **05.01.2000**
(21) Application number: 95914809.9
(22) Date of filing: 24.03.1995
(51) Int. Cl.: C07C 257/14, C07C 391/00, A61K 31/195, A61K 31/095

(54) **AMIDINO DERIVATIVES USEFUL AS NITRIC OXIDE SYNTHASE INHIBITORS**
AMIDINO-DERIVATIVE ALS NO-SYNTHESE-INHIBITOREN
DERIVES AMIDINO UTILES COMME INHIBITEURS DE LA SYNTHASE D'OXYDE NITRIQUE

(30) Priority: 24.03.1994 US 218160
(43) Date of publication of application: 08.01.1997
(73) Proprietor: G.D. SEARLE & CO., Chicago IL 60680-5110 (US)
(72) Inventor: CURRIE, Mark, G., St. Charles, MO 63304 (US); WEBBER, Keith, St. Peters, MO 63376 (US); TJOENG, Foe, S., Manchester, MO 63021 (US); FOK, Kam, F., St. Louis, MO 63146 (US)
(74) Representative: Weisert, Annekäte, Dipl.-Ing. Dr.-Ing.
(86) International application number: US9503589
(87) International publication number: WO9525717

(56) References cited:
- WO-A-93/13055
- WO-A-93/24126

## Description

The present invention relates to novel amidino derivates, pharmaceutical compositions containing these novel compounds, and to their use in therapy, in particular their use as nitric oxide synthase inhibitors.

### Related Art

It has been known since the early 1980's that the vascular relaxation brought about by acetycholine is dependent on the presence of the endothelium and this activity was ascribed to a labile humoral factor termed endothelium-derived relaxing factor (EDRF). The activity of nitric oxide (NO) as a vasodilator has been known for well over 100 years and NO is the active component of amylnitrite, glyceryltrinitrite and other nitrovasodilators. The recent identification of EDRF as NO has coincided with the discovery of a biochemical pathway by which NO is synthesized from the amino acid L-arginine by the enzyme NO synthase.

NO is the endogenous stimulator of the soluble guanylate cyclase and is involved in a number of biological actions in addition to endothelium-dependent relaxation including cytotoxicity of phagocytic cells and cell-to-cell communication in the central nervous system (see Moncada et al, Biochemical Pharmacology, 38, 1709-1715 (1989) and Moncada et al, Pharmacological Reviews, 43, 109-142 (1991). It is now thought that excess NO production may be involved in a number of conditions, particularly conditions which involve systemic hypertension such as toxic shock and therapy with certain cytokines.

The synthesis of NO from L-arginine can be inhibited by the L-arginine analogue, L-N-monomethyl-arginine (L-NMMA) and the therapeutic use of L-NMMA for the treatment of toxic shock and other types of systemic hypertension has been proposed (WO 91/04024 and GB-A-2240041). The therapeutic use of certain other NO synthase inhibitors apart from L-NMMA for the same purpose has also been proposed in WO 91/04024 and in EP-A-0446699.

It has recently become apparent that there are at least two types of NO synthase as follows:
(I) a constitutive, Ca⁺⁺/calmodulin dependent enzyme that releases NO in response to receptor or physical stimulation.
(ii) a Ca⁺⁺ independent enzyme which is induced after activation of vascular smooth muscle, macrophages, endothelial cells, and a number of other cells by endotoxin and cytokines. Once expressed this inducible NO synthase synthesizes NO for long periods.

The NO released by the constitutive enzyme acts as a transduction mechanism underlying several physiological responses. The NO produced by the inducible enzyme is a cytotoxic molecule for tumor cells and invading microorganisms. It also appears that the adverse effects of excess NO production, in particular pathological vasodilation and tissue damage, may result largely from the effects of NO synthesized by the inducible NO synthase.

Some of the NO synthase inhibitors proposed for therapeutic use so far, and in particular L-NMMA, are non-selective in that they inhibit both the constitutive and the inducible NO synthase. Use of such a non-selective NO synthase inhibitor requires that great care be taken in order to avoid the potentially serious consequences of over-inhibition of the constitutive NO-synthase including hypertension and possible thrombosis and tissue damage. In particular, in the case of the therapeutic use of L-NMMA for the treatment of toxic shock it has been recommended that the patient must be subject to continuous blood pressure monitoring throughout the treatment. Thus, while non-selective NO synthase inhibitors have therapeutic utility provided that appropriate precautions are taken, NO synthase inhibitors which are selective in the sense that they inhibit the inducible NO synthase to a considerably greater extent than the constitutive NO synthase would be of even greater therapeutic benefit and easier to use.

WO 93/13055, WO93/24126 and U.S. Patent No. 5,132,453 disclose compounds that inhibit nitric oxide synthesis and preferentially inhibit the inducible isoform of nitric oxide synthase.

### Summary of the Invention

The present invention provides amidino derivatives of the formula (I): 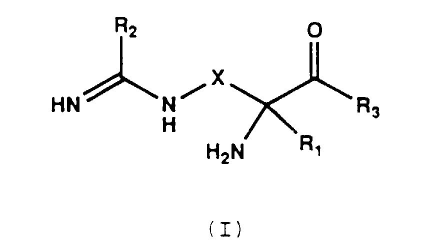 salts, pharmaceutically acceptable esters and prodrugs thereof, wherein
X is C₁-C₁₀ alkyl, C₂-C₁₀ alkenyl, C₂-C₁₀ alkynyl, -(CH₂)ₖQ(CH₂)l- where k is 2 or 3, l is 1 or 2 and Q is O, Se, SiY₂ where Y is C₁-C₁₀alkyl, S(O)_{Z} where is 0, 1 or 2, NR where R is hydrogen or C₁-C₁₀alkyl,
and wherein all said radicals may optionally be substituted by one or more substituents selected from C₁-C₁₀ alkyl, halogen, and trifluoroalkyl,
R₁ is hydrogen (when Q is Se), C₁-C₁₀ alkyl or hydroxyalkyl,
R₂ is C₁-C₁₀ alkyl, C₂-C₁₀ alkenyl, C₂-C₁₀ alkynyl or haloalkyl, and
R₃ is amino, alkylamine, amino acid, hydroxy, C₁-C₄ alkoxy, tetrazole, or tetrazoloamine.

The invention further relates to pharmaceutical compositions comprising a compound of formula (I). Compounds and compositions defined above have usefulness as inhibitors of nitric oxide synthase. These compounds also prefentially inhibit the inducible form over the constitutive form of nitric oxide synthase and in the most prefered form inhibit the inducible form over the constitutive form of nitric oxide synthase by at least 3 fold.

Conditions in which there is an advantage in inhibiting NO production from L-arginine include systemic hypotension associated with septic and/or toxic shock induced by a wide variety of agents; therapy with cytokines such as TNF, IL-1 and IL-2; and as an adjuvant to short term immunosuppression in transplant therapy.

There is also a growing body of evidence that NO may be involved in the degeneration of cartilage which takes place in certain conditions such as arthritis and it is also known that NO synthesis is increased in rheumatoid arthritis. Accordingly, further conditions in which there is an advantage in inhibiting NO production from L-arginine include autoimmune and/or inflammatory conditions affecting the joints, for example arthritis.

Still further conditions in which there is an advantage in inhibiting NO production from L-arginine include inflammatory bowel disease, cardiovascular ischemia, diabetes, cerebral ischemia and other CNS disorders mediated by NO.

### Detailed Description of the Invention

A preferred embodiment of the present invention is a compound of the formula (I): 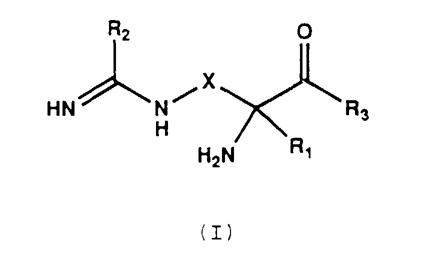 and salts, and pharmaceutically acceptable ester and prodrugs thereof, wherein:
X is C₁-C₁₀alkyl, C₂-C₁₀ alkenyl, C₂-C₁₀ alkynyl, -(CH₂)ₖQ(CH₂)l- where k is 2 or 3, l is 1 or 2 and Q is O, Se, SiY₂ where Y is C₁-C₁₀ alkyl, S(O)_{z} where z is 0, 1 or 2, NR where R is hydrogen or C₁-C₁₀ alkyl,
wherein all said radicals may optionally be substituted by one or more substituents selected from C₁-C₁₀ alkyl, halogen and trifluoroalkyl,
R₁ is methyl;
R₂ is C₁-C₁₀ alkyl, C₂-C₁₀ alkenyl, C₂-C₁₀ alkynyl and haloalkyl and
R₃ is amino, alkylamine, natural amino acid, hydroxy, C₂-C₄ alkoxy, tetrazole, or tetrazoloamine.

Another preferred embodiment of the present invention is a compound of the formula (I) : 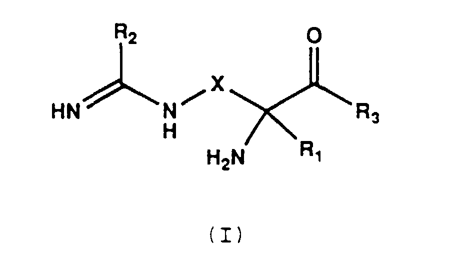 and salts, and pharmaceutically acceptable ester and prodrugs thereof, wherein:
X is a C₁-C₆ alkyl, C₁-C₆ alkenyl, C₁-C₆ alkynyl of 1 to 6 carbon atoms, -(CH₂)ₖQ(CH₂)ₗ- where k is 2 or 3, l is 1 or 2 and Q is O, Se, SiY₂ where Y is C₁-C₁₀ alkyl, S(O)z where z is 0, 1 or 2, or NR where R is H or C₁-C₁₀ alkyl;
wherein all said radicals may optionally be substituted by one or more substituents selected from C₁-C₁₀ alkyl, halogen and trifluoroalkyl,
R₁ is methyl;
R₂ is C₁-C₄ alkyl, C₂-C₄ alkenyl, C₂-C₄ alkynyl, and haloalkyl of 1 to 4 carbon atoms; and
R₃ is an amino, alkylamine of 1 to 4 carbon atoms, hydroxy, alkoxy of 1 to 4 carbon atoms, tetrazole, tetrazoloamine, or natural amino acid.

Still another preferred embodiment of the present invention is a compound of the formula (I) as shown above wherein X is an alkylene group having 3 to 5 carbon atoms and which may optionally be substituted by one or more C₁₋₃ alkyl; a group of formula -(CH₂)ₖQ(CH₂)ₗ- where k is 2 or 3, l is 1 or 2 and Q is O, Se, S(O)z where z is 0, 1 or 2;
R1 is methyl;
R₂ is an alkyl radical of 1 to 4 carbon atoms; and
R₃ is an amine, alkylamine of 1 to 4 carbon atoms, hydroxy, an alkoxy group of 1 to 4 carbon atoms.

The present invention includes compounds of formula (I) in the form of salts, in particular acid addition salts. Suitable salts include those formed with both organic and inorganic acids. Such acid addition salts will normally be pharmaceutically acceptable although salts of non-pharmaceutically acceptable salts may be of utility in the preparation and purification of the compound in question. Thus, preferred salts include those formed from hydrochloric, hydrobromic, sulphuric, citric, tartaric, phosphoric, lactic, pyruvic, acetic, succinic, oxalic, fumaric, maleic, oxaloacetic, methanesulphonic, ethanesulphonic, p-toluenesulphonic, benzenesulphonic and isethionic acids. Salts of the compounds of formula (I) can be made by reacting the appropriate compound in the form of the free base with the appropriate acid.

While it may be possible for the compounds of formula (I) to be administered as the raw chemical, it is preferable to present them as a pharmaceutical formulation. According to a further aspect, the present invention provides a pharmaceutical formulation comprising a compound of formula (I) or a pharmaceutically acceptable salt or solvate thereof, together with one or more pharmaceutically acceptable carriers thereof and optionally one or more other therapeutic ingredients. The carrier(s) must be "acceptable" in the sense of being compatible with the other ingredients of the formulation and not deleterious to the recipient thereof.

The formulations include those suitable for oral, parenteral (including subcutaneous, intradermal, intramuscular, intravenous and intraarticular), rectal and topical (including dermal, buccal, sublingual and intraocular) administration although the most suitable route may depend upon for example the condition and disorder of the recipient. The formulations may conveniently be presented in unit dosage form and may be prepared by any of the methods well known in the art of pharmacy. All methods include the step of bringing into association a compound of formula (I) or a pharmaceutically acceptable salt or solvate thereof ("active ingredient") with the carrier which constitutes one or more accessory ingredients. In general, the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations for parenteral administration include aqueous and non-aqueous sterile injection solutions which may contain antioxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example, saline, water-for-injection, immediately prior to use. Extemporaneous injection solutions and suspensions may be prepared from sterile powders, granules and tablets of the kind previously described.

Formulations for rectal administration may be presented as a suppository with the usual carriers such as cocoa butter or polyethylene glycol.

Formulations for topical administration in the mouth, for example buccally or sublingually, include lozenges comprising the active ingredient in a flavored basis such as sucrose and acacia or tragacanth, and pastilles comprising the active ingredient in a basis such as gelatin and glycerin or sucrose and acacia.

Preferred unit dosage formulations are those containing an effective dose, as hereinbelow recited, or an appropriate fraction thereof, of the active ingredient.

It should be understood that in addition to the ingredients particularly mentioned above, the formulations of this invention may include other agents conventional in the art having regard to the type of formulation in question, for example those suitable for oral administration may include flavoring agents.

The compounds of the invention may be administered orally or via injection at a dose of from 0.1 to 500 mg/kg per day. The dose range for adult humans is generally from 5mg to 2g/day. Tablets or other forms of presentation provided in discrete units may conveniently contain an amount of compound of the invention which is effective at such dosage or as a multiple of the same, for instance, units containing 5mg to 500mg, usually around 10mg to 200mg.

The compounds of formula (I) are preferably administered orally or by injection (intravenous or subcutaneous). The precise amount of compound administered to a patient will be the responsibility of the attendant physician. However, the dose employed will depend on a number of factors, including the age and sex of the patient, the precise disorder being treated, and its severity. Also, the route of administration may vary depending on the condition and its severity.

As utilized herein, the term "C₁-C₁₀ alkyl", alone or in combination, means an acyclic alkyl radical containing from 1 to 10, preferably from 1 to 8 carbon atoms and more preferably 1 to 6 carbon atoms. Examples of such radicals include methyl, ethyl, n-propyl, isopropyl, n-butyl, isobutyl, sec-butyl, tert-butyl, pentyl, iso-amyl, hexyl, octyl and the like.

The term "C₂-C₁₀ alkenyl" refers to an unsaturated acyclic hydrocarbon radical in so much as it contains at least one double bond. Such radicals containing from 2 to 10 carbon atoms, preferably from 2 to 8 carbon atoms and more preferably 2 to 6 carbon atoms. Examples of suitable alkenyl radicals include propylenyl, buten-1-yl, isobutenyl, pentenylen-1-yl, 2-2-methylbuten-1-yl, 3-methylbuten-1-yl, hexen-1-yl, hepten-1-yl, and octen-1-yl, and the like.

The term "C₂-C₁₀ alkynyl" refers to an unsaturated acyclic hydrocarbon radicals in so much as it contains one or more triple bonds, such radicals containing 2 to 10 carbon atoms, preferably having from 2 to 8 carbon atoms and more preferably having 2 to 6 carbon atoms. Examples of suitable alkynyl radicals include ethynyl, propynyl, butyn-1-yl, butyn-2-yl, pentyn-1-yl, pentyn-2-yl, 3-methylbutyn-1-yl, exyn-1-yl, hexyn-2-yl, hexyn-3-yl, 3,3-dimethylbutyn-1-yl radicals and the like.

The term "halogen" means fluorine, chlorine, bromine or iodine.

The term amino acid means an amino alkanoic wherein the amino can be postioned anywhere on the alkyl group. The alkyl is as is defined above.

The term "prodrug" refers to a compound that is made more active *in vivo.*

As used herein, reference to "treatment" of a patient is intended to include prophylaxis.

Disclosed is a general synthetic processes useful in the preparation of the compounds of the present invention.

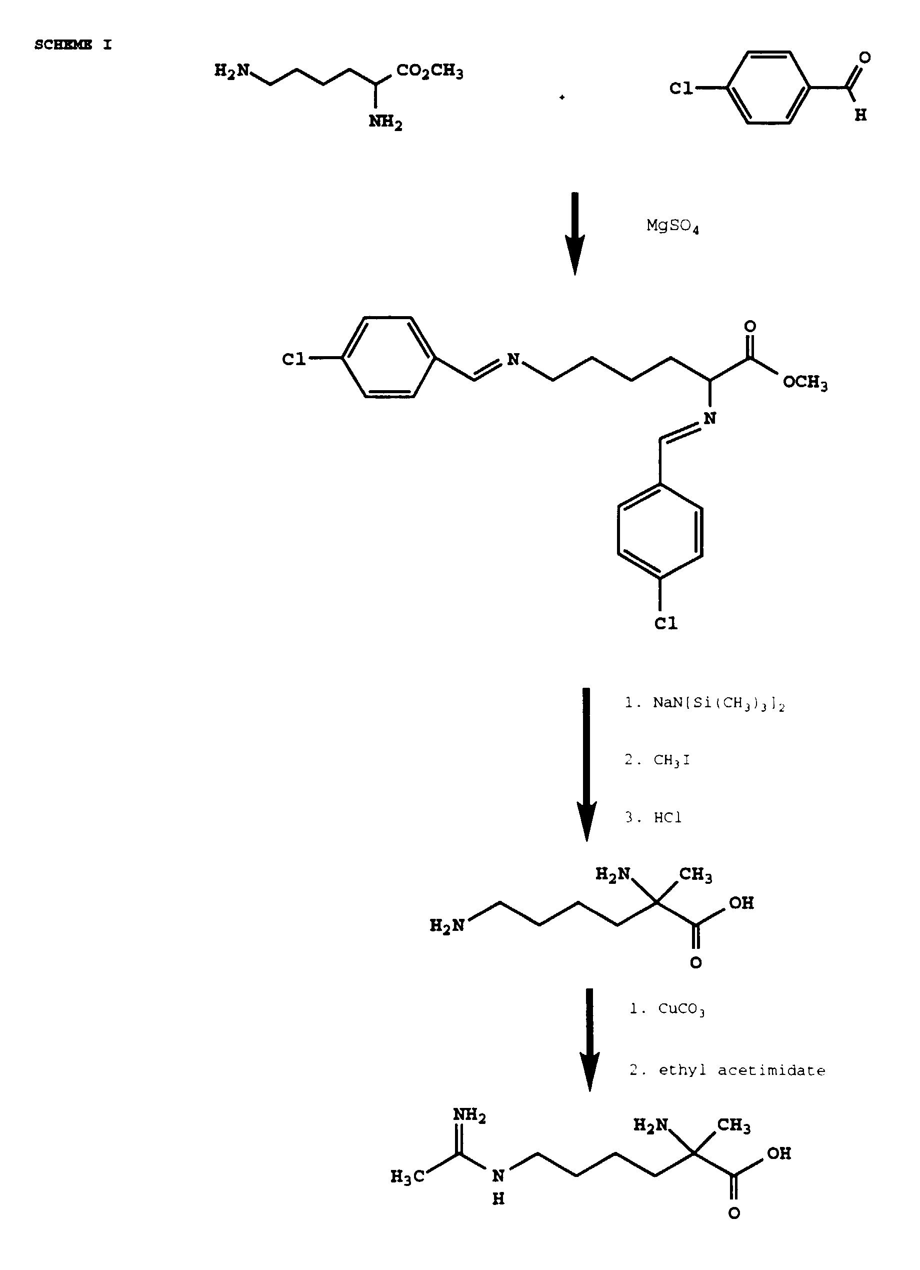

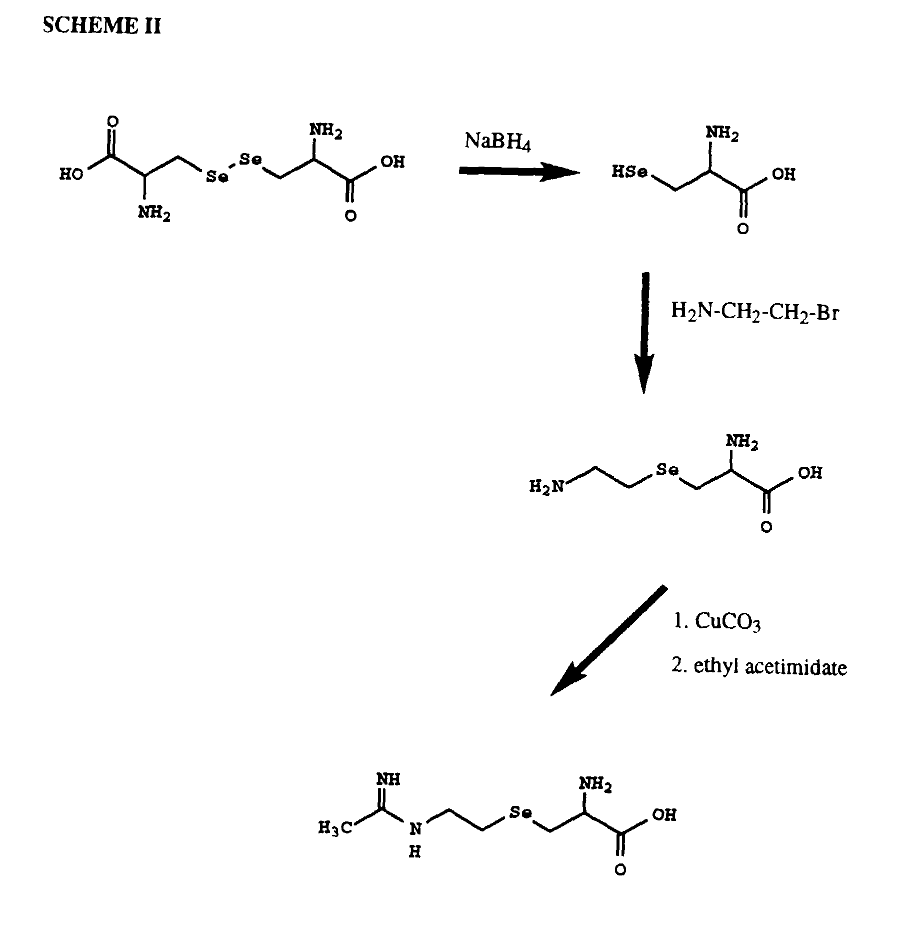

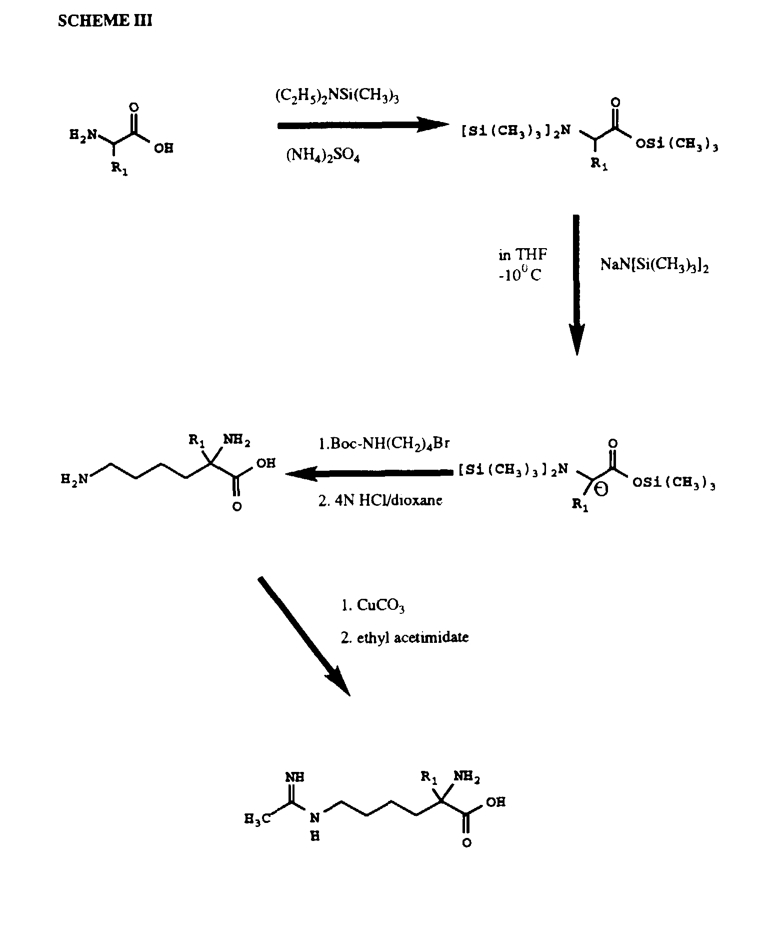

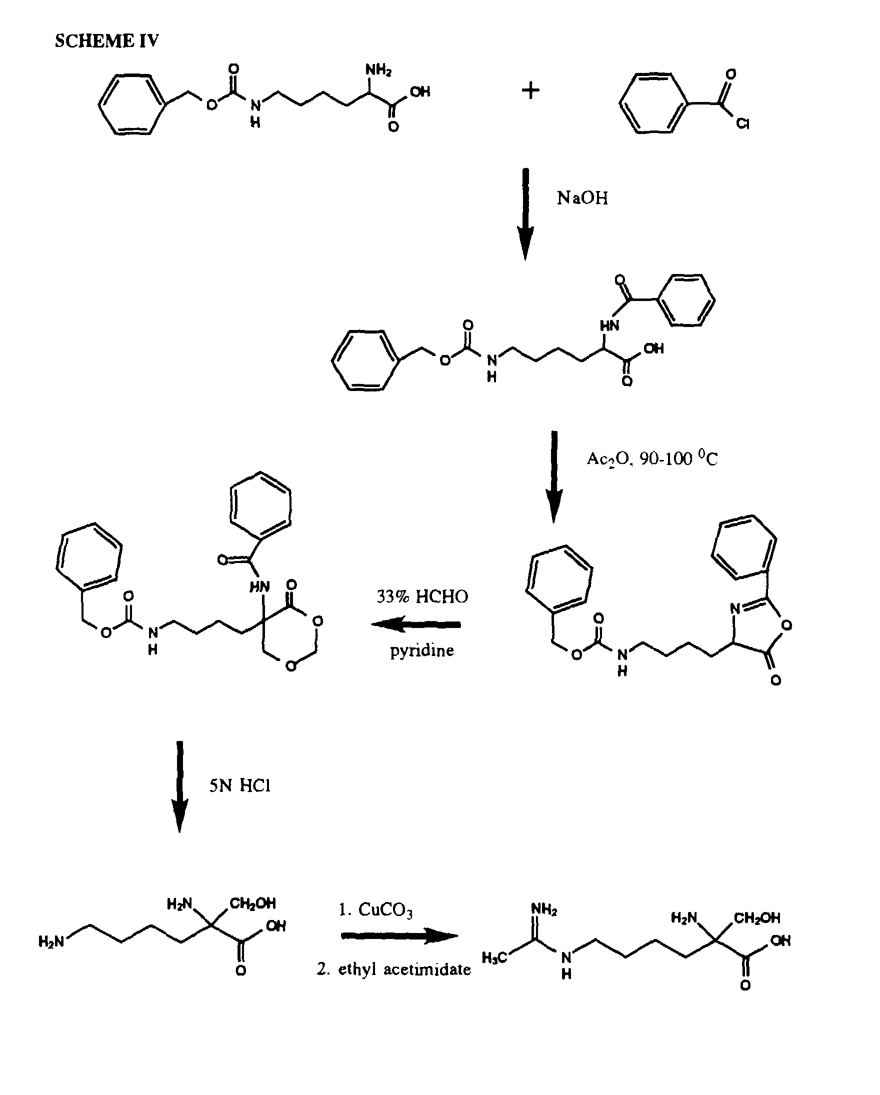

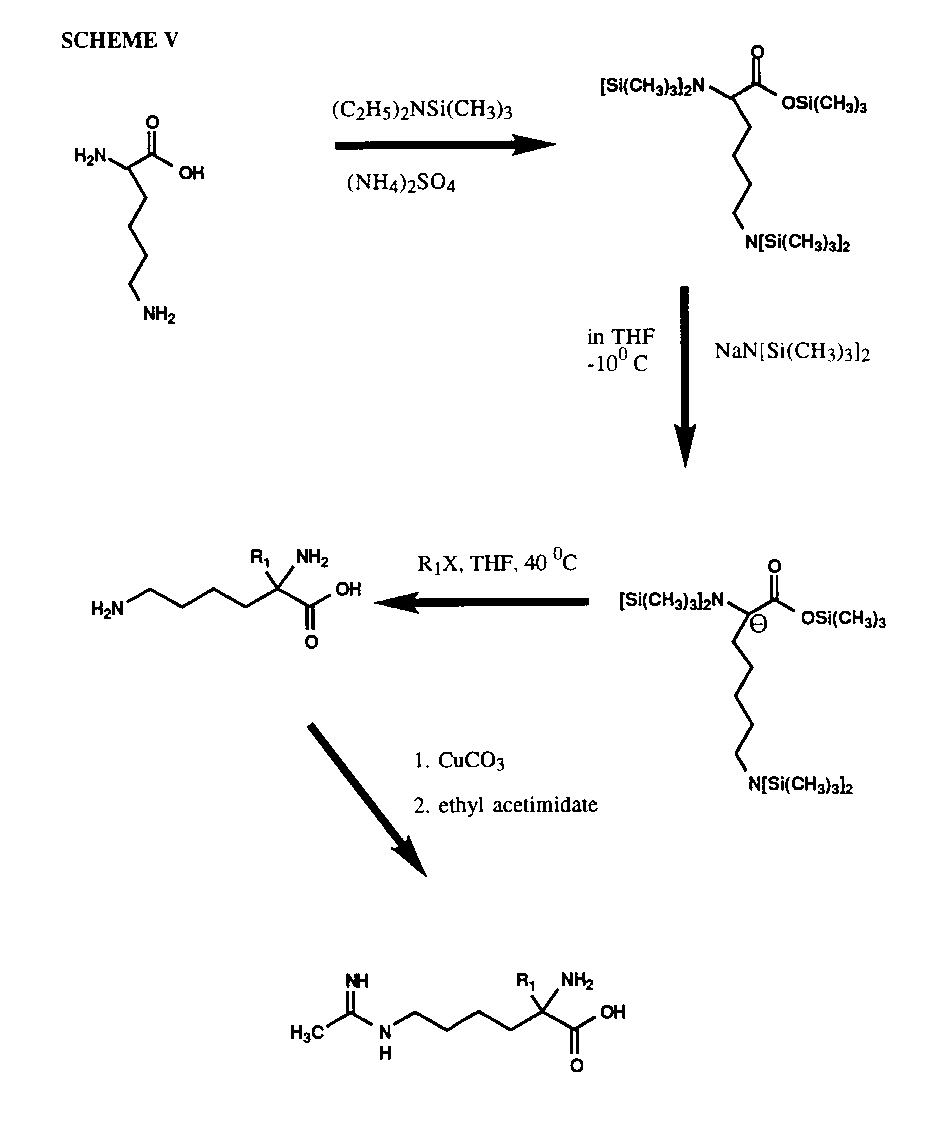

The invention is further illustrated by the following examples:

### EXAMPLE 1

### α-Methyl-Nδ-iminoethyl-ornithine

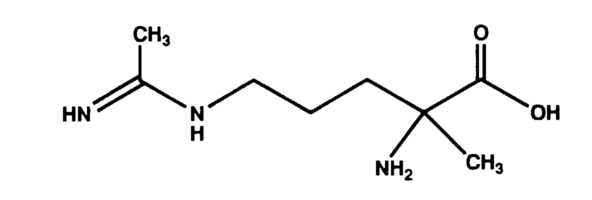

α-Methyl-D,L-ornithine hydrochloride (250 mg; 1.37 mmoles purchased from Sigma) was dissolved in 5 ml of water and 0.5 g of cupric carbonate was added. The mixture was stirred at 60 °C for 1 hr and filtered. The pH of the filtrate was adjusted to pH 9.5 with 0.5 N NaOH solution. Ethyl acetimidate hydrochloride (178 mg; 1.45 mmoles) was added and the pH was maintained at 9.0-9.5 for one hour. The solution was then adjusted to a pH of 7.5 and kept at room temperature for 12 hours. The solution was acidified with 1 N HCl to pH 4 and applied to a Dowex 50 X 4 (hydrogen form). The column was washed with water and then with 10 % pyridine. α-Methyl-Nδ-iminoethylornithine was eluted from the column with 1 N NH⁴OH. The ninhydrin positive fractions were combined and lyophilized. The residue was dissolved in 0.5 N of aqueous hydrochloric acid and re-lyophilized. α-Methyl-Nδ-iminoethyl-ornithine hydrochloride appears as white solids. (MH⁺ = 188); 1H-NMR (D20): d 1.4 (s, 3H); 1.45-1.9 (m, 4 H); 2.15 (s, 3 H); 3.3 (t, 2 H).

### EXAMPLE 2

### α-Methyl-Nε-iminoethyl-lysine

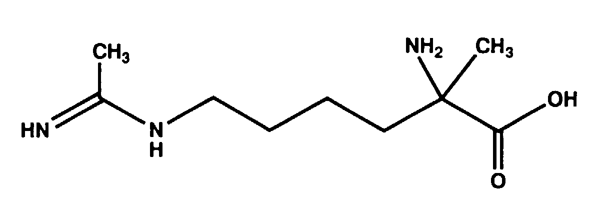

A suspension of lysine ethyl ester dihydrochloride (33 g; 0.14 mole) and MgSO₄ (34 g; 0.28 moles) in a solution of 4-chloro-benzaldehyde (39 g; 0.28 moles) and acetonitrile (500 ml) was stirred while N,N-diisopropylethylamine (36 g; 0.28 moles) was added in portions over 1/2 h. The mixture was stirred for 12 h, filtered, concentrated to a small volume, and diluted with 500 ml of ethyl ether. The ether solution was washed with 0.1% aqueous NaHCO₃, aqueous 2 N NaOH containing 2g/100 ml of NH₂OH.HCl, again with 0.1% aqueous NaHCO₃ and saturated aqueous NaCl. After drying with MgSO₄ and removal of the solvent in vacuo, ethyl N, N'-di(4-chloro-phenylmethylene)-L-alanine was obtained as a clear liquid. The liquid was triturated with hexanes and the resulting solid was washed with hexanes for several times. This partially purified intermediate was dissolved in 200 ml of THF and stirred in an acetone/dry ice bath. Sodium bis-(trimethylsilyl)amide in THF (11 ml, 1 M solution) was added dropwisely over 30 min. After one hour, methyl iodide (0.8 g; 13 mmoles) in THF was added dropwisely. The reaction mixture was slowly warmed up to room temperature and stirred overnight. The mixture was diluted with water, and extracted with ethyl ether. The ether extract was washed with 0.1% aqueous NaHCO3 and saturated aqueous NaCl and concentrated to yield crude ethyl N.N'-di(4-chloro-phenylmethylene)-α-methyl-D,L-alanine (M + H= 434). This material (4 g) dissolved in ethyl ether (100 ml) was stirred vigorously with 1 N HCl (50 ml) for 2 h, the layer was separated and the aqueous phase was washed with ethyl ether. The aqueous solution was further acidified by the addition of concentrated HCl to 6 N and was heated to reflux for 16 h. The solution was cooled to room temperature, and rotary evaporated to dryness. The residue was dissolved in water and applied to a Dowex 50 X 4 (hydrogen form).The column was washed with water, and then 10% pyridine. α-Methyl-D,L-lysine, (MH⁺ H = 161) was eluted from the column with 1 M NH4OH. α-Methyl-D,L-lysine (300 mg) was dissolved in water and 0.5 g of cupric carbonate was added. The suspension was stirred at 600 C for 1 h and filtered. The filtrate was adjusted to pH 9.5 and ethyl acetimidate was added portionwise in 15 min. The pH was maintained at 9.0 to 9.5. After 1 h of stirring at room temperature, the solution was adjusted to pH 7 and the stirring was continued overnight. α-Methyl-Nε-iminoethyl-lysine was purified by Dowex 50X4 (hydrogen form) chromatography similarly described for α-Methyl-Nδ-iminoethyl-ornithine. MH⁺ = 202; 1H-NMR (D₂0): d 1.4 (s, 3H); 1.5-1.8 (m, 6 H); 2.05 (s, 3 H); 3.1 (t, 2 H).

### EXAMPLE 3

### Nε-iminoethyl-aminoethylselenocysteine

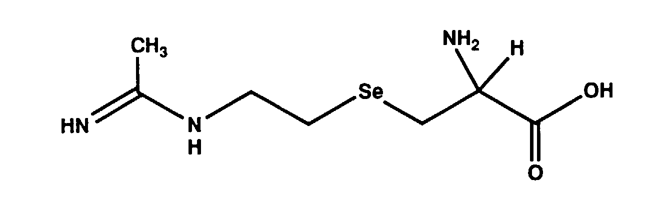

Seleno-D,L-cystine (117 mg; 0.5 mmoles, purchased from Sigma) was suspended in 15 ml of nitrogen gas-purged water. Sodium borohydride (38 mg; 1 mole) was added. The reaction mixture became clear in a few minutes. After 2 h at room temperature, 2-bromoethylamine HCl (1.2 g; 6 mmoles) was added and the reaction mixture was stirred for 12 h. The reaction was applied on to a Dowex 50 X 4 (hydrogen form) column. The column was washed with water and 10% pyridine. 2-Aminoethyl-D,L-seleno-cysteine was eluted with 1 M NH4OH and, subsequently, treated with cupric carbonate and ethyl acetimidate as described for Nδ-iminoethyl-ornithine. Nε-iminoethylaminoethylselenocysteine was obtained as pale yellow solids. MH⁺ = 254.

### EXAMPLE 4

### α-Hydroxymethyl-Nε-iminoethyl-lysine

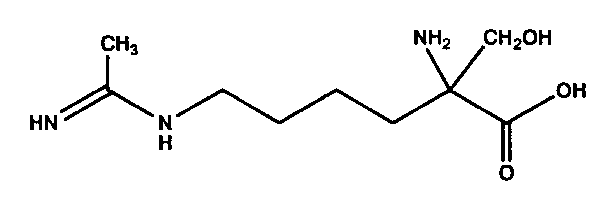

To an ice-cold stirred mixture of Nε-Cbz-L-lysine (14 g; 0.05 moles, purchased from Sigma) in 2.5 N NaOH (24 ml), benzoyl chloride (10 g) was added gradually. The pH of the solution was maintained at 10.5-10.9 by addition of 2 N NaOH. The mixture was stirred at room temperature for 1 h and filtered. The filtrate was extracted with a small amount of ethyl acetate and the organic layer was dried over sodium sulfate. The solid was removed by filtration and the filtrate was evaporated to dryness. The remaining crude oily Nε-Cbz-Nα-benzoyl-lysine (6 g) was heated at 90-100° C with acetic anhydride (100 ml) for 30 min. The mixture was then evaporated. The residue was dissolved in pyridine and treated with aqueous formaldehyde (35% solution, Fisher). The mixture was stirred for 8 hr, then diluted. The reaction mixture was kept at 100C overnight the precipitated crude material was hydrolyzed in boiling 5 N HCl during 5 h. The reaction mixture was cooled and filtered before being evaporated. The solid residue was dissolved in water and passed through Dowex 50 X 4 (hydrogen form) column. α-Hydroxymethyl-D,L-lysine (MH⁺ = 177) was eluted with 1 N NH₄OH and, subsequently, treated with cupric carbonate and ethyl acetimidate to form α-hydroxymethyl-Nε-iminoethyl-lysine similarly described for α-Methyl-Nδ-iminoethyl-ornithine. MH⁺ = 218. 1H-NMR (D₂0): d 1.1-1.8 (m, 6H); 2.1 (s, 3H); 3.1 (t, 2H); 3.6-3.9 (q(a,b), 2H).

The activity of the above listed compounds as NO synthase inhibitors have been determined in the following assays:

### Citrulline Assay for Nitric Oxide Synthase

Nitric oxide synthase activity was measured by monitoring the conversion of [³H]-arginine to [³H]-citrulline. Mouse inducible nitric oxide synthase (miNOS) was prepared from an extract of LPS-treated RAW 264.7 cells and partially purified by DEAE-Sepharose chromatography. Rat brain constitutive nitric oxide synthase (rnNOS) was prepared from an extract of rat cerebellum and partially purified by DEAE-Sepharose chromatography. Enzyme and inhibitors were incubated at 37°C for 15 minutes in a reaction volume of 100 µL with the following components added to start the reaction: 50 mM Tris (pH 7.6), 1 mg/ml bovine serum albumin, 1 mM DTT, 2 mM CaCl₂, 10 µM FAD, 10 µM tetrahydrobiopterin, 30 µM L-arginine containing L-[2,3-³H]-arginine at 300 cpm/pmole and 1 mM NADPH. For constitutive NOS, 50 nM calmodulin was also added. The reaction was terminated by addition of cold stop buffer containing 10 mM EGTA, 100 mM HEPES, pH 5.5 and 1 mM citrulline. [³H]-Citrulline was separated by chromatography on Dowex 50W X-8 cation exchange resin and radioactivity determined with a liquid scintillation counter.

Recombinant human inducible nitric oxide synthase (hiNOS) was extracted from Sf9 insect cells (infected with recombinant baculoviruses encoding a cDNA for human inducible nitric oxide synthase which has been isolated and purified from a lambda Zapll cDNA library made from RNA isolated from a colon sample from a patient with ulcerative colitis) and partially purified by DEAE-chromatography.

### Raw Cell Nitrite Assay

RAW 264.7 cells are plated to confluency on a 96-well tissue culture plate grown overnight (17h) in the presence of LPS to induce NOS. A row of 3-6 wells were left untreated and served as controls for subtraction of nonspecific background. The media was removed from each well and the cells are washed twice with Krebs-Ringers-Hepes (25mM, pH 7.4) with 2 mg/ml glucose. The cells are then placed on ice and incubated with 50 µL of buffer containing L-arginine (30 µM) +/- inhibitors for lh. The assay is initiated by warming the plate to 37°C in a water bath for lh. Production of nitrite by intracellular iNOs is linear with time. To terminate the cellular assay, the plate of cells is placed on ice and the nitrite-containing buffer removed and analyzed for nitrite using a previously published fluorescent determination for nitrite. All values are the average of triplicate wells and are compared to a background-subtracted induced set of cells (100% value).

LPS is the abbreviation for lipopolysaccharide.

**TABLE I**

| Compound Cell | hiNos | miNOS | rnNOS | Raw |
|---|---|---|---|---|
| [µM] | IC₅₀ [µM] | | IC₅₀ | |
| Example 1 | 73.5 | 34.9 | 355.9 | 25.0 |
| | | | | |
| Example 2 | 26.6 | 7.2 | 192.8 | 52.5 |
| | | | | |
| Example 3 | 35.5 | 5.0 | 229.0 | 0.37 |
| | | | | |
| Example 4 | 39% Inh. @ 10 µM | | 8.6% Inh. @ 10 µM | |

## Claims

1. A Compound having the formula: 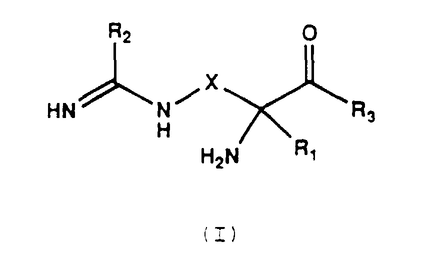 salts, pharmaceutically acceptable esters and prodrugs thereof, wherein
X is C₁-C₁₀ alkyl, C₂-C₁₀ alkenyl, C₂-C₁₀ alkynyl, (CH₂)ₖQ(CH₂)l- where k is 2 or 3, l is 1 or 2 and Q is O, Se, SiY₂ where Y is C₁-C₁₀alkyl, S(O)_{z} where z is 0, 1 or 2, NR where R is hydrogen or C₁-C₁₀ alkyl,
and wherein all said adicals may optionally be substituted by one or more substituents selected from C₁-C₁₀ alkyl, halogen, and trifluoroalkyl,
R₁ is hydrogen (when Q is Se), C₁-C₁₀ alkyl or hydroxyalkyl,
R₂ is C₁-C₁₀ alkyl, C₂-C₁₀ alkenyl, C₂-C₁₀ alkynyl or haloalkyl ; and
R₃ is amino, alkylamine, amino acid, hydroxy, C₁-C₄ alkoxy, tetrazole, or tetrazoloamine.

2. The compound as recited in claim 1 wherein
X is C₁-C₁₀alkyl, C₂-C₁₀ alkenyl, C₂-C₁₀ alkynyl, -(CH₂)ₖQ(CH₂)l- where k is 2 or 3, l is 1 or 2 and Q is O, Se, SiY₂ where Y is C₁-C₁₀ alkyl, S(O)_{z} where z is O, 1 or 2, NR where R is hydrogen or C₁-C₁₀ alkyl,
wherein all said radicals may optionally be substituted by one or more substituents selected from C₁-C₁₀ alkyl, halogen and trifluoroalkyl,
R₁ is methyl.
R₂ is C₁-C₁₀ alkyl, C₂-C₁₀ alkenyl, C₂-C₁₀ alkynyl and haloalkyl and
R₃ is amino, alkylamine, natural amino acid, hydroxy, C₂-C₄ alkoxy, tetrazole, or tetrazoloamine.

3. The compound as recited in claim 1 wherein
X is a C₁-C₆ alkyl, C₁-C₆ alkenyl, C₁-C₆ alkynyl of 1 to 6 carbon atoms, -(CH₂)ₖQ(CH₂)ₗ- where k is 2 or 3, l is 1 or 2 and Q is O, Se, SiY₂ where Y is C₁-C₁₀ alkyl, S(O)z where z is 0, 1 or 2, or NR where R is H or C₁-C₁₀ alkyl;
wherein all said radicals may optionally be substituted by one or more substituents selected from C₁-C₁₀ alkyl, halogen and trifluoroalkyl,
R₁ is methyl;
R₂ is C₁-C₄ alkyl, C₂-C₄ alkenyl, C₂-C₄ alkynyl, and haloalkyl of 1 to 4 carbon atoms; and
R₃ is an amino, alkylamine of 1 to 4 carbon atoms, hydroxy, alkoxy of 1 to 4 carbon atoms, tetrazole, tetrazoloamine, or natural amino acid.

4. The compound as recited in claim 1 wherein
X is an alkylene group having 3 to 5 carbon atoms and which may optionally be substituted by one or more C₁₋₃ alkyl; a group of formula -(CH₂)ₖQ(CH₂)ₗ- where k is 2 or 3, l is 1 or 2 and Q is O, Se, S(O)z where z is 0, 1 or 2;
R1 is methyl;
R₂ is an alkyl radical of 2 to 4 carbon atoms; and
R₃ is an amino, alkylamine of 1 to 4 carbon atoms, hydroxy, an alkoxy group of 1 to 4 carbon atoms.

5. The compound as recited in claim 1 selected from the group consisting of α-Methyl-Nδ-iminoethyl-ornithine, α-Methyl-Nε-iminoethyl-lysine, α-Methyl-Nε-iminoethylaminoethyl-selenocysteine and α-Hydroxymethyl-Nε-iminoethyl-lysine.

6. A pharmaceutical composition comprising a compound as recited in claims 1 to 5 together with a pharmaceutically acceptable carrier.

7. Use of the compound as is recited in Claim 1, 2, 3, 4 or 5 for preparing a medicament for inhibiting nitric oxide synthesis in a subject in need of such inhibition.

## Patentansprüche

1. Verbindung der Formel: 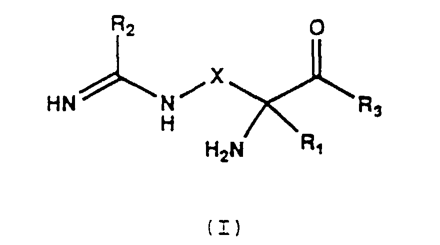 Salze, pharmazeutisch annehmbare Ester und Prodrugs davon, worin
X C₁-C₁₀-Alkyl, C₂-C₁₀-Alkenyl, C₂-C₁₀-Alkynyl, -(CH₂)ₖQ(CH₂)ₗ-, worin k gleich 2 oder 3 ist, l gleich 1 oder 2 ist und Q für O, Se, SiY₂ steht, wobei Y C₁-C₁₀-Alkyl bedeutet, S(O)_{z}, wobei z gleich 0, 1 oder 2 ist, NR, wobei R für Wasserstoff oder C₁-C₁₀-Alkyl steht, bedeutet,
und worin alle Reste gegebenenfalls durch einen oder mehrere Substituenten, ausgewählt aus C₁-C₁₀-Alkyl, Halogen und Trifluoralkyl, substituiert sein können,
R₁ für Wasserstoff (wenn Q gleich Se ist), C₁-C₁₀-Alkyl oder Hydroxyalkyl steht,
R₂ für C₁-C₁₀-Alkyl, C₂-C₁₀-Alkenyl, C₂-C₁₀-Alkynyl oder Haloalkyl steht und
R₃ für Amino, Alkylamin, einen Aminosäurerest, Hydroxy, C₁-C₄-Alkoxy, Tetrazol oder Tetrazolamin steht.

2. Verbindung nach Anspruch 1, worin
X C₁-C₁₀-Alkyl, C₂-C₁₀-Alkenyl, C₂-C₁₀-Alkynyl, -(CH₂)ₖQ(CH₂)ₗ-, worin k gleich 2 oder 3 ist, l gleich 1 oder 2 ist und Q für O, Se, SiY₂ steht, wobei Y C₁-C₁₀-Alkyl bedeutet, S(O)_{z}, wobei z gleich 0, 1 oder 2 ist, NR, wobei R für Wasserstoff oder C₁-C₁₀-Alkyl steht, bedeutet,
worin alle Reste gegebenenfalls durch einen oder mehrere Substituenten, ausgewählt aus C₁-C₁₀-Alkyl, Halogen und Trifluoralkyl, substituiert sein können,
R₁ für Methyl steht,
R₂ für C₁-C₁₀-Alkyl, C₂-C₁₀-Alkenyl, C₂-C₁₀-Alkynyl oder Haloalkyl steht und
R₃ für Amino, Alkylamin, einen natürlichen Aminosäurerest, Hydroxy, C₂-C₄-Alkoxy, Tetrazol oder Tetrazolamin steht.

3. Verbindung nach Anspruch 1, worin
X C₁-C₆-Alkyl, C₁-C₆-Alkenyl, C₁-C₆ Alkynyl mit 1 bis 6 Kohlenstoffatomen, -(CH₂)ₖQ(CH₂)ₗ-, worin k gleich 2 oder 3 ist, l gleich 1 oder 2 ist und Q für O, Se, SiY₂ steht, wobei Y C₁-C₁₀-Alkyl bedeutet, S(O)_{z}, wobei z gleich 0, 1 oder 2 ist, oder NR, wobei R für Wasserstoff oder C₁-C₁₀-Alkyl steht, bedeutet,
worin alle Reste gegebenenfalls durch einen oder mehrere Substituenten, ausgewählt aus C₁-C₁₀-Alkyl, Halogen und Trifluoralkyl substituiert sein können,
R₁ für Methyl steht,
R₂ für C₁-C₄-Alkyl, C₂-C₄ Alkenyl, C₂-C₄ Alkynyl und Haloalkyl mit 1 bis 4 Kohlenstoffatomen steht; und
R₃ für Amino, Alkylamin mit 1 bis 4 Kohlenstoffatomen, Hydroxy, Alkoxy mit 1 bis 4 Kohlenstoffatomen, Tetrazol, Tetrazolamin oder einen natürlichen Aminosäurerest steht.

4. Verbindung nach Anspruch 1, worin
X eine Alkylengruppe mit 3 bis 5 Kohlenstoffatomen, welche gegebenenfalls durch eine oder mehrere C₁-C₃-Alkylgruppen substituiert sein kann, eine Gruppe der Formel -(CH₂)ₖQ(CH₂)ₗ-, worin k gleich 2 oder 3 ist, l gleich 1 oder 2 ist und Q für O, Se oder S(O)_{z} steht, wobei z gleich 0, 1 oder 2 ist, bedeutet;
R₁ für Methyl steht;
R₂ für einen Alkylrest mit 1 bis 4 Kohlenstoffatomen steht; und
R₃ für Amino, Alkylamin mit 1 bis 4 Kohlenstoffatomen, Hydroxy oder eine Alkoxygruppe mit 1 bis 4 Kohlenstoffatomen steht.

5. Verbindung nach Anspruch 1, ausgewählt aus der Gruppe, bestehend aus α-Methyl-Nδ-iminoethyl-ornithin, α-Methyl-Nε-iminoethyl-lysin, α-Methyl-Nε-iminoethyl-aminoethylselenocystein und α-Hydroxymethyl-Nε-iminoethyl-lysin.

6. Pharmazeutische Zubereitung, umfassend eine Verbindung nach Anspruch 1 bis 5 zusammen mit einem pharmazeutisch annehmbaren Träger.

7. Verwendung der Verbindung nach Anspruch 1, 2, 3, 4 oder 5 zur Herstellung eines Medikaments zur Inhibierung der Stickstoffmonoxidsynthese bei einer Versuchsperson, bei der eine solche Inhibition erforderlich ist.

## Revendications

1. Composé ayant la formule : 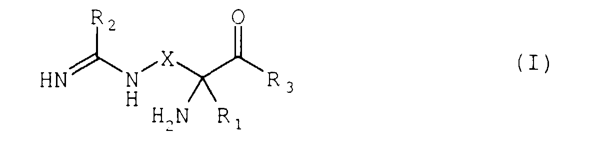 sels, esters pharmaceutiquement acceptables et prodrogues de celui-ci, dans lesquels :
X est alkyle en C₁ à C₁₀, alcényle en C₂ à C₁₀, alcynyle en C₂ à C₁₀, -(CH₂)ₖQ(CH₂)ₗ- où k vaut 2 ou 3, l vaut 1 ou 2 et Q est O, Se, SiY₂ où Y est alkyle en C₁ à C₁₀, S(O)_{z} où z vaut 0, 1 ou 2, NR où R est hydrogène ou alkyle en C₁ à C₁₀,
et dans lesquels l'ensemble desdits radicaux peuvent être le cas échéant substitués par un ou plusieurs substituants choisis parmi alkyle en C₁ à C₁₀, halogène et trifluoralkyle,
R₁ est hydrogène (lorsque Q est Se), alkyle en C₁ à C₁₀ ou hydroxyalkyle,
R₂ est alkyle en C₁ à C₁₀, alcényle en C₂ à C₁₀, alcynyle en C₂ à C₁₀ ou haloalkyle, et
R₃ est amino, alkylamino, acide aminé, hydroxy, alcoxy en C₁ à C₄, tétrazole ou tétrazolamine.

2. Composé selon la revendication 1, dans lequel :
X est alkyle en C₁ à C₁₀, alcényle en C₂ à C₁₀, alcynyle en C₂ à C₁₀, -(CH₂)ₖQ(CH₂)ₗ- où k vaut 2 ou 3, l vaut 1 ou 2 et Q est O, Se, SiY₂ où Y est alkyle en C₁ à C₁₀, S(O)_{z} où z vaut 0, 1 ou 2, NR où R est hydrogène ou alkyle en C₁ à C₁₀,
et dans lesquels l'ensemble desdits radicaux peuvent être le cas échéant substitués par un ou plusieurs substituants choisis parmi alkyle en C₁ à C₁₀, halogène et trifluoralkyle,
R₁ est méthyle,
R₂ est alkyle en C₁ à C₁₀, alcényle en C₂ à C₁₀, alcynyle en C₂ à C₁₀ ou haloalkyle, et
R₃ est amino, alkylamino, acide aminé naturel, hydroxy, alcoxy en C₂ à C₄, tétrazole ou tétrazolamine.

3. Composé selon la revendication 1, dans lequel :
X est alkyle en C₁ à C₆, alcényle en C₁ à C₆, alcynyle en C₁ à C₆ ayant de 1 à 6 atomes de carbone, -(CH₂)ₖQ(CH₂)ₗ- où k vaut 2 ou 3, l vaut 1 ou 2 et Q est O, Se, SiY₂ où Y est alkyle en C₁ à C₁₀, S(O)_{z} où z vaut 0, 1 ou 2, NR où R est H ou alkyle en C₁ à C₁₀,
dans lesquels l'ensemble desdits radicaux peuvent être le cas échéant substitués par un ou plusieurs substituants choisis parmi alkyle en C₁ à C₁₀, halogène et trifluoralkyle,
R₁ est méthyle,
R₂ est alkyle en C₁ à C₄, alcényle en C₂ à C₄, alcynyle en C₂ à C₄ ou haloalkyle ayant de 1 à 4 atomes de carbone, et
R₃ est amino, alkylamino ayant de 1 à 4 atomes de carbone, hydroxy, alcoxy ayant de 1 à 4 atomes de carbone, tétrazole, tétrazolamine ou acide aminé naturel.

4. Composé selon la revendication 1, dans lequel :
X est un groupe alkylène ayant de 3 à 5 atomes de carbone qui peut le cas échéant être substitué par un ou plusieurs groupes alkyle en C₁ à C₃ ; un groupe de formule -(CH₂)ₖQ(CH₂)ₗ- où k vaut 2 ou 3, l vaut 1 ou 2 et Q est O, Se, S(O)_{z} où z vaut 0, 1 ou 2,
R₁ est méthyle,
R₂ est un radical alkyle ayant de 1 à 4 atomes de carbone, et
R₃ est un groupe amino, alkylamino ayant de 1 à 4 atomes de carbone, hydroxy, alcoxy ayant de 1 à 4 atomes de carbone.

5. Composé selon la revendication 1, choisi dans le groupe comprenant la α-méthyl-Nδ-iminoéthylornithine, la α-méthyl-Nε-iminoéthyllysine, la α-méthyl-Nε-iminoéthylaminoéthylsélénocystéine et la α-hydroxyméthyl-Nε-iminoéthyllysine.

6. Composition pharmaceutique comprenant un composé selon les revendications 1 à 5 ensemble avec un véhicule pharmaceutiquement acceptable.

7. Utilisation du composé selon la revendication 1, 2, 3, 4 ou 5 pour préparer un médicament destiné à inhiber la synthèse de l'oxyde nitrique chez un sujet nécessitant une telle inhibition.
